# EUROPÄISCHE PATENTSCHRIFT

(11) **EP 1 747 762 B1**
(45) Veröffentlichungstag und Bekanntmachung des Hinweises auf die Patenterteilung: **16.03.2011**
(21) Anmeldenummer: 06012708.1
(22) Anmeldetag: 21.06.2006
(51) Int. Cl.: A61B 18/14, A61B 17/28

(54) **Koagulationsklemme für chirurgische Zwecke**
Coagulation forceps for surgical applications
Pince de coagulation pour procedures chirurgicales

(30) Priorität: 26.07.2005 DE 102005034816
(43) Veröffentlichungstag der Anmeldung: 31.01.2007
(73) Patentinhaber: Aesculap AG, 78532 Tuttlingen (DE)
(72) Erfinder: Rothweiler, Christoph, 78166 Donaueschingen (DE); Weisshaupt, Dieter, 78194 Immendingen (DE); Keller, Anton, 78589 Dürbheim (DE)
(74) Vertreter: Hoeger, Stellrecht & Partner Patentanwälte

(56) Entgegenhaltungen:
- EP-A- 0 986 990
- EP-A- 1 568 330
- WO-A-00/06030
- US-A- 6 010 516
- US-A1- 2004 122 423
- US-B1- 6 887 240

## Beschreibung

Die Erfindung betrifft eine Koagulationsklemme für chirurgische Zwecke mit zwei gegeneinander bewegbaren Klemmbacken mit elektrisch leitenden und mit elektrisch isolierenden Bereichen, wobei die elektrisch isolierenden Bereiche durch zum jeweils anderen Klemmbacken weisende Vorsprünge auf einer elektrisch leitenden Grundfläche der Klemmbacken gebildet werden, wobei Anschläge vorgesehen sind, die die Bewegung der beiden Klemmbacken bei deren Annäherung in ihrer angenäherten Schließstellung so begrenzen, dass die Grundflächen im wesentlichen parallel zueinander und im Abstand zueinander angeordnet sind, wobei die Vorsprünge so lang und so angeordnet sind, dass die Vorsprünge eines Klemmbackens in der Schließstellung der Klemmbacken zwischen die Vorsprünge des anderen Klemmbackens eintauchen, wobei der Querschnitt der Vorsprünge im Überlappungsbereich der Vorsprünge der beiden Klemmbacken zum freien Ende der Vorsprünge hin abnimmt, und wobei die Vorsprünge so angeordnet und geformt sind, dass die von den Vorsprüngen freie Fläche in parallel zu den Grundflächen verlaufenden Ebenen in der Schließstellung der Klemmbacken über den gesamten Zwischenraum zwischen den Grundflächen der beiden Klemmbacken im wesentlichen gleich ist.

Derartige Koagulationsklemmen werden verwendet, um zwischen den Klemmbacken gefasstes Gewebe mittels eines zwischen den elektrisch leitenden Bereichen der beiden Klemmbacken fließenden Stromes zu behandeln, beispielsweise um in diesen Bereichen eine Koagulation zu erreichen. Eine bekannte Koagulationsklemme ist in der DE 102 31 369 A1 dargestellt.

In der EP 1 568 330 A1 ist eine Koagulationsklemme mit den vorstehend angegebenen Merkmalen beschrieben, bei der die Klemmbacken selbst aus elektrisch leitendem Material bestehen, auf die elektrisch isolierende Vorsprünge aufgesetzt sind.

Ausgehend von diesem Stand der Technik liegt dem Anmeldegegenstand die Aufgabe zugrunde, eine gattungsgemäße Koagulationsklemme anzugeben, die einen einfachen Aufbau hat und daher einfach herstellbar ist.

Diese Aufgabe wird bei einer Koagulationsklemme der eingangs beschriebenen Art erfindungsgemäß dadurch gelöst, dass die Klemmbacken und die Vorsprünge einstückig ausgebildet sind und die elektrisch leitfähige Grundfläche durch eine Metallfüllung einer oder mehrerer Vertiefungen in den Klemmbacken gebildet wird.

Beispielsweise können Klemmbacken Verwendung finden, die aus Keramik bestehen und die entsprechende Vorsprünge tragen, zwischen diesen Vorsprüngen ist eine Vertiefung im Klemmbacken mit Metall ausgefüllt, beispielsweise durch Ausgießen, und die außenseitige Grenzfläche dieser Metallfüllung bildet die elektrisch leitfähige Grundfläche zwischen den Vorsprüngen des Klemmbackens aus.

Durch die elektrisch isolierenden Vorsprünge kann ein Stromfluss nur in den Bereichen erfolgen, die von diesen Vorsprüngen frei sind, und diese freien Bereiche werden durch die spezielle Anordnung und Formgebung der Vorsprünge so gewählt, dass deren Querschnitt über den gesamten Zwischenraum zwischen den Koagulationsklemmen gleich ist, dass mit anderen Worten auch die Stromdichte über die gesamte Stromflussstrecke zwischen den elektrisch leitenden Grundflächen im wesentlichen gleich bleibt, es gibt also keine Bereiche, in denen die Stromdichte nach oben oder nach unten wesentlich von einem Mittelwert abweicht. Es gibt also längs des Stromweges zwischen den beiden Klemmbacken weder eine Einschnürung und damit eine erhöhte Stromdichte noch im Gegenteil eine übermäßige Ausbreitung des zu behandelnden Gewebes mit entsprechend abnehmender Stromdichte, die Vorsprünge zwingen das Gewebe in die seitlichen Räume zwischen den Vorsprüngen und erreichen somit einen gleichmäßigen Stromfluss über die gesamte Höhe des Zwischenraums zwischen den beiden elektrisch leitenden Grundflächen.

Die Abnahme des Querschnittes der Vorsprünge führt dazu, dass auch im Überlappungsbereich der für den Stromfluss zur Verfügung stehende Querschnitt im wesentlichen gleich bleibt wie außerhalb des Überlappungsbereiches, d.h. die Abnahme der Querschnittsfläche der Vorsprünge des einen Klemmbackens in Richtung auf den anderen Klemmbacken kompensiert im wesentlichen die entsprechende Zunahme des Querschnitts der Vorsprünge des anderen Klemmbackens, so dass über die Höhe des Zwischenraumes gesehen die für den Stromfluss zur Verfügung stehende, von den Vorsprüngen freie Fläche konstant bleibt.

Wenn dabei von "im wesentlichen gleichen Flächen" gesprochen wird, so umfasst dies exakt gleiche Flächen und auch Flächen, die sich nur geringfügig voneinander unterscheiden. So können beispielsweise die von den Vorsprüngen eingenommenen Flächen und die von den Vorsprüngen freien Flächen um bis zu 20 % voneinander abweichen, insbesondere gilt dies im Überlappungsbereich der Vorsprünge von gegenüberliegenden Klemmbacken. In diesem Bereich ist die Zahl der Vorsprünge durch die Überlappung größer, und entsprechend muss die Querschnittsfläche der Vorsprünge abnehmen, um annähernd eine Gleichheit der von den Vorsprüngen eingenommenen und der von den Vorsprüngen freien Fläche herzustellen. Dies ist aufgrund der speziellen Formgebung der Vorsprünge nicht immer exakt erreichbar, beispielsweise dann, wenn die Vorsprünge am freien Ende ballig ausgebildet sind. Der Effekt einer gleichmäßigen Stromflussstrecke wird jedoch optimal erreicht, wenn die Flächen möglichst gleich groß sind.

Es ist vorteilhaft, wenn die von den Vorsprüngen freie Fläche in parallel zu den Grundflächen verlaufenden Ebenen mindestens so groß ist wie die von den Vorsprüngen eingenommene Fläche so daß für den Stromfluß und damit für die Behandlung des zwischen den Vorsprüngen eingeklemmten Gewebes eine genügend große Fläche zur Verfügung steht. Dies ist wesentlich, um eine homogene Behandlung des Gewebes mit dem Strom zu erreichen.

Die Vorsprünge können beispielsweise im Überlappungsbereich konisch oder auch ballig ausgebildet sein.

Außerhalb des Überlappungsbereiches ist es günstig, wenn die Vorsprünge zylindrisch ausgebildet sind, insbesondere kreiszylindrisch.

In einem anderen Ausführungsbeispiel können die Vorsprünge als sich über die Breite der Klemmbacken erstreckende Querrippen ausgebildet sein. Diese können bei einem bevorzugten Ausführungsbeispiel im Querschnitt halbkreisförmig ausgebildet sein.

Es ist vorteilhaft, wenn der Abstand von zwei Bereichen der von den Vorsprüngen freien Fläche, die durch einen Vorsprung voneinander getrennt sind, maximal 1,5 mm beträgt. Dies bedeutet, daß bei zapfenförmigen Vorsprüngen deren Durchmesser maximal 1,5 mm beträgt, bei querrippenförmigen Vorsprüngen deren Breite maximal 1,5 mm. Dadurch ist sichergestellt, daß Teile des eingeklemmten Gewebes, die von Strom unmittelbar durchflossen werden, maximal 1,5 mm voneinander entfernt sind, und dies genügt, um eine homogene Strombehandlung zu erreichen. Größere Abstände der stromdurchflossenen Fläche könnten zu sogenannten Abschattungen führen, d.h. zu Bereichen im Gewebe, die nicht ausreichend von dem Strom erreicht werden.

Es ist günstig, wenn die Vorsprünge aus Keramik bestehen. Bei einer anderen Ausführungsform können die Vorsprünge jedoch auch aus einem thermoplastischen Kunststoff bestehen, beispielsweise aus Ketonen oder Imiden, insbesondere aus Polyetheretherketon. Es handelt sich dabei um sogenannte Hochleistungsthermoplaste, die sterilisierbar sind.

Die nachfolgende Beschreibung bevorzugter Ausführungsformen der Erfindung dient im Zusammenhang mit der Zeichnung der näheren Erläuterung. Es zeigen:
- Figur 1:: eine perspektivische Ansicht der Klemmbacken eines ersten be- vorzugten Ausführungsbeispiels einer Koagulationsklemme in geöffneter Stellung;
- Figur 2:: eine Draufsicht auf einen Klemmbacken mit zusätzlicher An- gabe der Lage der Vorsprünge des anderen Klemmbackens bei in der Schließstellung stehenden Klemmbacken;
- Figur 3:: eine Schnittansicht längs Linie 3-3 mit im Überlappungsbereich konisch ausgebildeten Vorsprüngen;
- Figur 4:: eine Ansicht ähnlich Figur 3 mit im Überlappungsbereich ballig ausgebildeten Vorsprüngen;
- Figur 5:: eine Ansicht ähnlich Figur 1 bei einem weiteren bevorzugten Ausführungsbeispiel einer Koagulationsklemme 1 mit einer abweichenden Verteilung der Vorsprünge der beiden Klemmbacken;
- Figur 6:: eine Ansicht ähnlich Figur 3 mit sich über die gesamte Höhe des Zwischenraums zwischen zwei Klemmbacken erstreckenden, konischen Vorsprüngen;
- Figur 7:: eine Ansicht ähnlich Figur 1 mit Vorsprüngen an den Klemmbacken in Form von im Querschnitt halbkreisförmigen Querrippen und
- Figur 8:: eine Seitenansicht der Klemmbacken der Figur 7 in Schließstellung.

Die Erfindung wird nachstehend am Beispiel eines Koagulationsinstrumentes 1 beschrieben, das zangenförmig ausgebildet ist, bei dem also zwei Schenkel 2, 3 in einem Gelenk 4 schwenkbar miteinander verbunden sind, die an ihren Enden jeweils einen Klemmbacken 5, 6 tragen. Diese Klemmbacken 5, 6 können durch Verschwenken der Schenkel 2, 3 gegeneinander von einer Offenstellung, in der sie auseinandergeschwenkt sind, in eine Schließstellung verschwenkt werden, in der sie im wesentlichen parallel zueinander stehen.

Es versteht sich aber, daß das Koagulationsinstrument nicht zwingend zangenförmig ausgebildet sein muß, es könnte auch eine pinzettenförmige Ausgestaltung gewählt werden. Auch ist es nicht unbedingt notwendig, daß die Klemmbacken 5, 6 zum Überführen aus der Offenstellung in die Schließstellung gegeneinander verschwenkt werden, es wäre auch eine Bewegung möglich, in der die Klemmbacken parallel zu sich selbst verschoben werden. Wesentlich ist lediglich, daß die Klemmbacken 5, 6 aus einer voneinander entfernten Offenstellung in eine einander angenäherte Schließstellung bewegt werden und in der Schließstellung die Klemmbacken 5, 6 im wesentlichen parallel zueinander liegen.

Bei dem in der Zeichnung dargestellten Koagulationsinstrument weisen die beiden Klemmbacken 5, 6 eine im wesentlichen rechteckige, am freien Ende abgerundete Form auf, die einander zugewandten Innenseiten der Klemmbakken 5, 6 sind im wesentlichen eben und bilden eine Grundfläche 7 bzw. 8 aus.

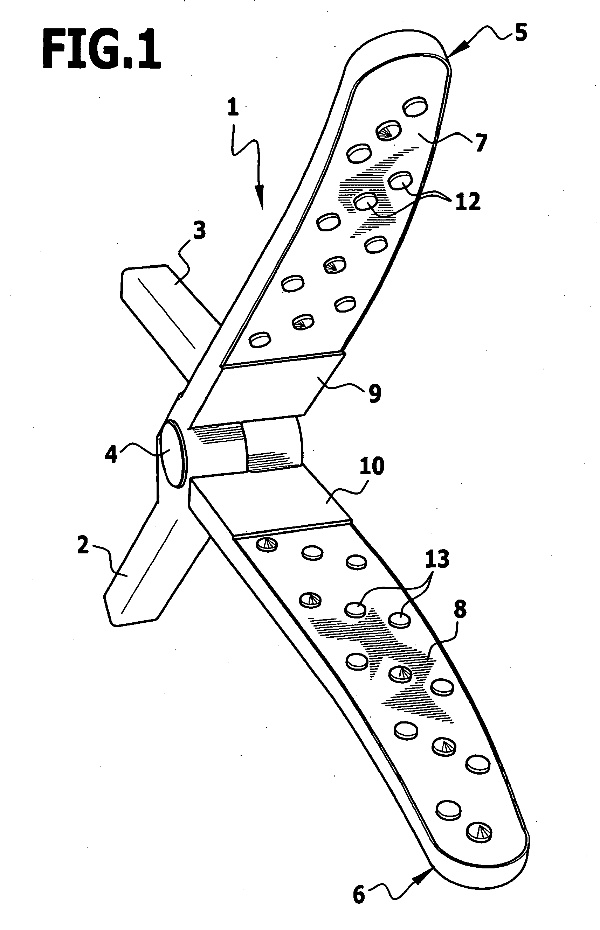

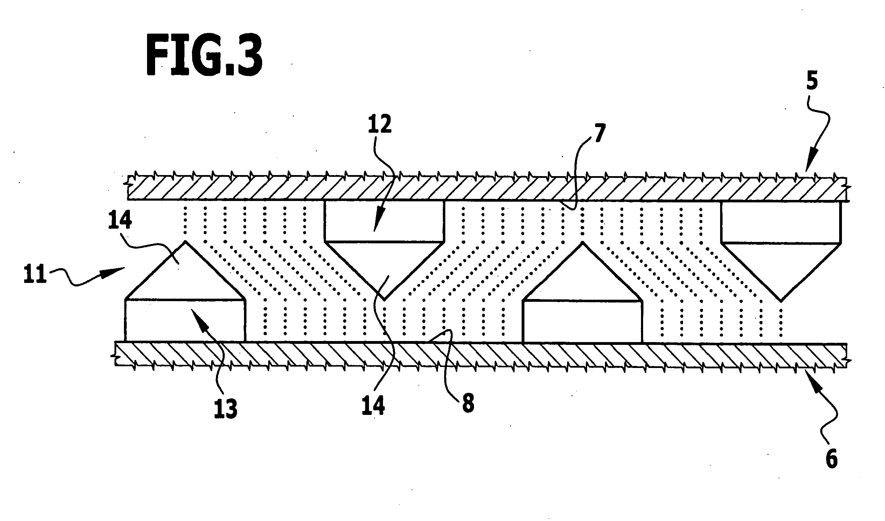

Bei dem Ausführungsbeispiel der Figur 1 sind im gelenknahen Bereich dieser Grundflächen 7, 8 Stufen 9, 10 vorgesehen, die beim Schließen der Klemmbacken 5, 6 aneinander zur Anlage kommen und damit die Schließbewegung begrenzen. In der Schließstellung, in der diese Stufen 9, 10 aneinander anliegen, verlaufen die Grundflächen 7, 8 der beiden Klemmbacken 5,6 im wesentlichen parallel und im Abstand zueinander, so daß zwischen ihnen ein Zwischenraum 11 verbleibt (Figur 3).

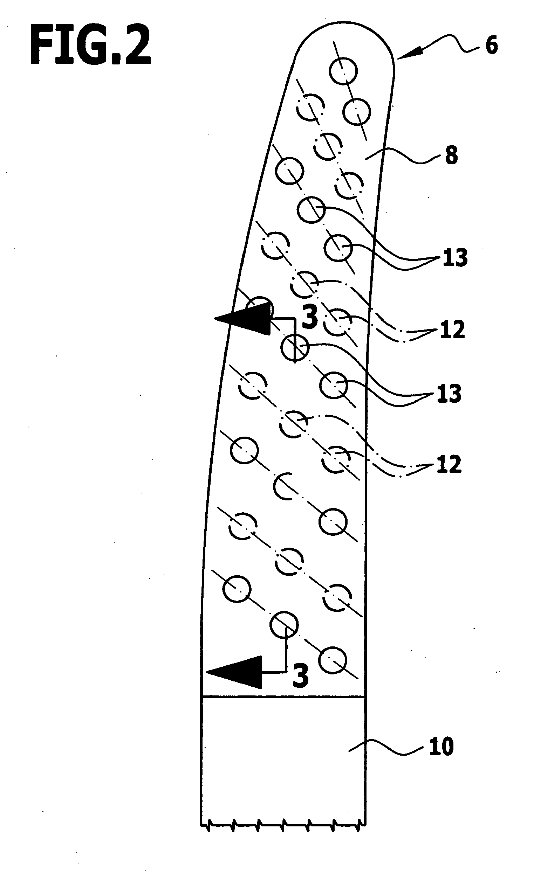

Von den Grundflächen 7, 8 stehen Vorsprünge 12 bzw. 13 hervor, die in Richtung auf den jeweils anderen Klemmbacken verlaufen und die im wesentlichen gleichmäßig über die Grundflächen 7 bzw. 8 verteilt sind, so daß zwischen ihnen ein im wesentlichen gleicher Abstand eingehalten ist. Die Vorsprünge der beiden Klemmbacken 5, 6 sind dabei so seitlich gegeneinander versetzt, daß sich die Vorsprünge 12 des Klemmbackens 5 und die Vorsprünge 13 des Klemmbackens 6 nicht gegenüberstehen, sondern daß die Vorsprünge des einen Klemmbackens im wesentlichen zwischen den Vorsprüngen des anderen Klemmbackens verlaufen (Figur 2).

Die Grundflächen 7, 8 sind elektrisch leitend ausgebildet, die Vorsprünge 12, 13 bestehen aus elektrisch nicht leitendem Material, insbesondere aus Keramik. Die Vorsprünge 12, 13 sind dabei mit den Klemmbacken 5 bzw. 6 einstückig aus Keramik geformt. Die Klemmbacken 5, 6 weisen zwischen den Vorsprüngen 12 eine Vertiefung oder einen Hohlraum auf, der mit einem metallischen Werkstoff ausgespritzt oder ausgegossen ist, dieser metallische Werkstoff bildet dann mit einer ebenen Oberseite die elektrisch leitende Grundfläche 7 bzw. 8 aus, die den gesamten Bereich zwischen den Vorsprüngen 12, 13 ausfüllt.

Bei einem ersten bevorzugten Ausführungsbeispiel sind die Vorsprünge 12, 13 kreiszylindrisch geformt und enden in Form eines Konuses 14. Die Vorsprünge 12, 13 haben dabei eine Länge, die kleiner ist als der Abstand zwischen den Grundflächen 7, 8 in der Schließstellung der Klemmbacken 5, 6, die jedoch so groß ist, daß die Vorsprünge des einen Klemmbackens zwischen die Vorsprünge des anderen Klemmbackens eintauchen. Die Anordnung ist dabei so gewählt, daß im Überlappungsbereich der Vorsprünge 12, 13 diese die Form eines Konus 14 aufweisen, außerhalb des Überlappungsbereichs sind sie jedoch zylindrisch geformt. Dadurch wird erreicht, daß die freie Fläche zwischen den Vorsprüngen 12, 13 in der Schließstellung der Klemmbacken 5, 6 in parallel zu den Grundflächen 7, 8 verlaufenden Ebenen über die gesamte Höhe des Zwischenraumes 11 im wesentlichen gleich ist. Diese freie Fläche ergibt sich nämlich aus der Differenz der jeweiligen Grundfläche 7, 8 und der Querschnittsfläche der Vorsprünge 12, 13 in der entsprechenden Ebene. Im Über lappungsbereich ist zwar die Zahl der Vorsprünge größer, durch die konische Ausbildung ist jedoch ihr Querschnitt gemindert, und zwar im wesentlichen so, daß die Summe der Querschnittsflächen der Vorsprünge der Querschnittsfläche entspricht, die die Vorsprünge außerhalb des Überlappungsbereiches einnehmen.

Durch diese Gleichheit der zur Verfügung stehenden Fläche zwischen den Vorsprüngen kann ein Gewebe, welches in der Schließstellung zwischen den Klemmbacken 5, 6 gehalten wird, von einem Strom, der von einer Grundfläche zur anderen fließt, mit im wesentlichen über den gesamten Stromweg gleicher Stromdichte durchflossen und damit gleichförmig behandelt, z.B. koaguliert, werden. Einschnürungen oder ausgedehnte Bereiche sind vermieden und damit Stromspitzen und zu niedrige Stromdichten. Über die gesamte Höhe des Zwischenraumes 11 und über dessen gesamte Ausdehnung ergibt sich somit eine gleichförmige Behandlung des zwischen den Klemmbacken gehaltenen Gewebes. Dies ist in Figur 3 durch die punktiert eingetragenen Stromwege symbolisiert, die im wesentlichen parallel zueinander den Zwischenraum durchsetzen.

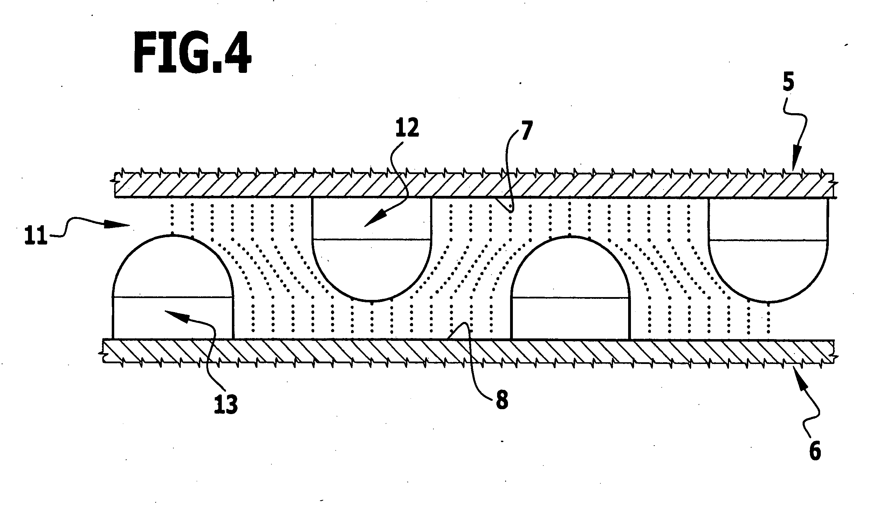

Während bei dem Ausführungsbeispiel der Figur 3 die Vorsprünge 12, 13 an ihren freien Enden konisch ausgebildet sind, kann ein ähnlicher Effekt erreicht werden, wenn gemäß der Ausgestaltung der Figur 4 die Vorsprünge 12, 13 zum freien Ende hin ballig ausgebildet sind. Auch in diesem Falle ist die Abnahme der Querschnittsfläche eines Vorsprunges des einen Klemmbackens in Richtung auf den anderen Klemmbacken etwa gleich groß wie die entsprechende Zunahme der Querschnittsfläche des Vorsprungs des anderen Klemmbackens, so daß auch im Überlappungsbereich dem Behandlungsstrom im wesentlichen eine über die gesamte Höhe des Zwischenraumes gleiche Fläche zur Verfügung steht.

Beim Ausführungsbeispiel der Figur 1 sind die Vorsprünge 12, 13 auf beiden Klemmbacken 5, 6 im wesentlichen gleichförmig über die gesamte Fläche verteilt.

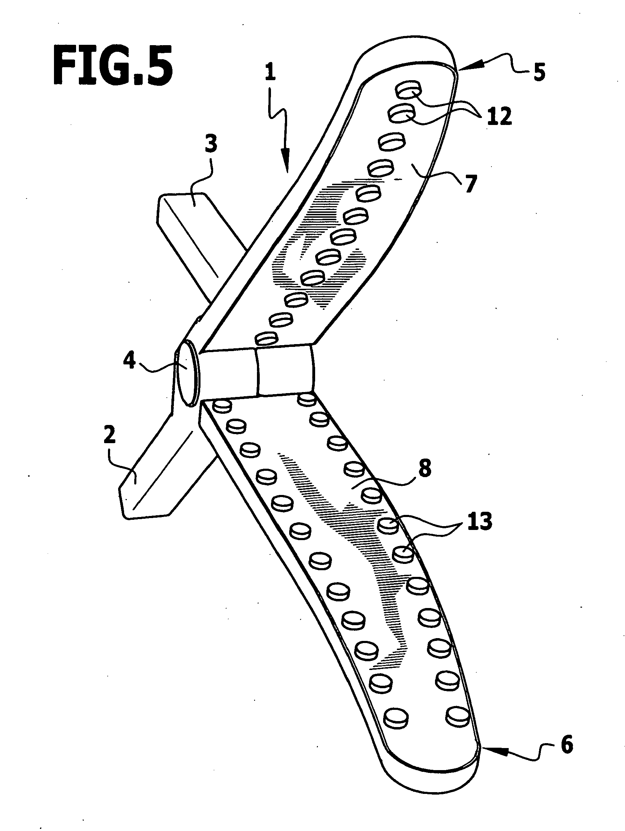

Im Gegensatz dazu ist beim Ausführungsbeispiel der Figur 5 vorgesehen, daß am unteren Klemmbacken 6 an dessen äußerem Rand jeweils eine Reihe von Vorsprüngen 13 angeordnet ist, am oberen Klemmbacken 5 dagegen nur eine mittige Reihe von Vorsprüngen. Trotz der möglicherweise unterschiedlichen Anzahl von Vorsprüngen an den beiden Klemmbacken kann aber die Bedingung nach im wesentlichen gleichen Flächen, die von einem Vorsprung eingenommen werden oder die von einem Vorsprung frei sind, im Bereich innerhalb der Vorsprünge 13 weitgehend erfüllt werden, so daß auch mit dieser Anordnung eine homogene Strombeaufschlagung des Gewebes möglich ist.

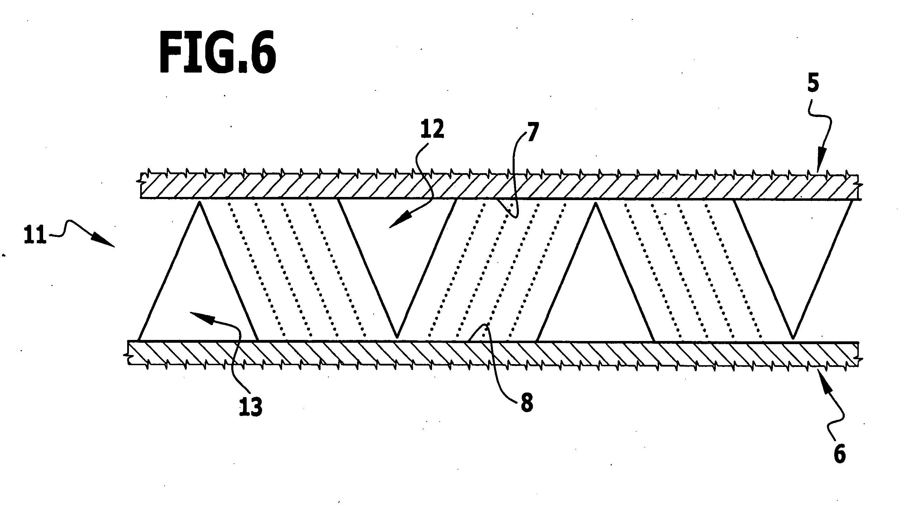

In Figur 6 ist ein abgewandeltes Ausführungsbeispiel dargestellt, bei dem die Vorsprünge 12, 13 über ihre gesamte Höhe konisch ausgebildet sind und sich jeweils bis zur Grundfläche des anderen Klemmbackens erstrecken. In diesem Falle kompensieren sich die Querschnittszunahmen und Querschnittsabnahmen der Vorsprünge 12 bzw. 13 in Richtung von einem Klemmbacken zum anderen, so daß auch in diesem Falle über die gesamte Höhe des Zwischenraumes 11 im wesentlichen gleiche Flächen für den Stromdurchfluß zur Verfügung stehen.

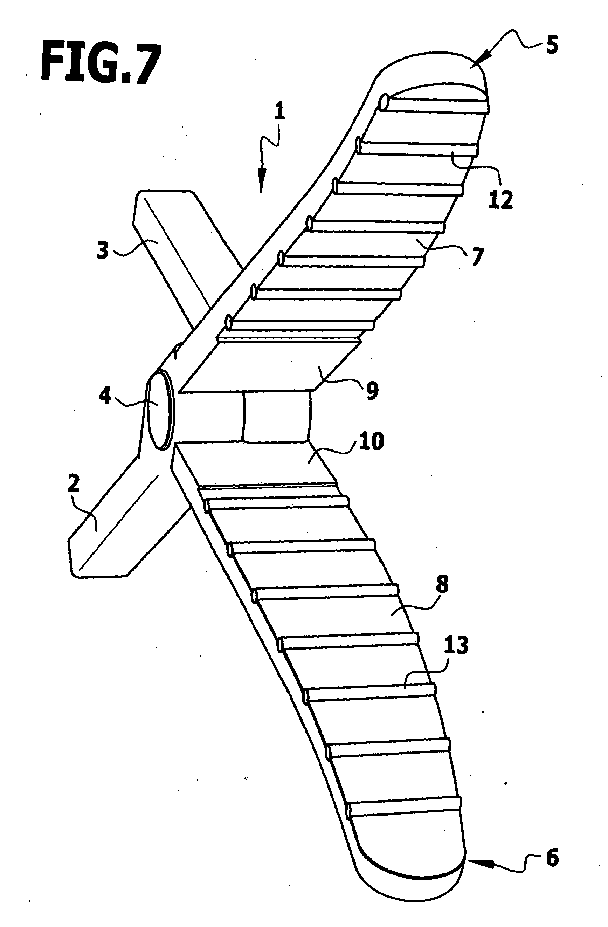

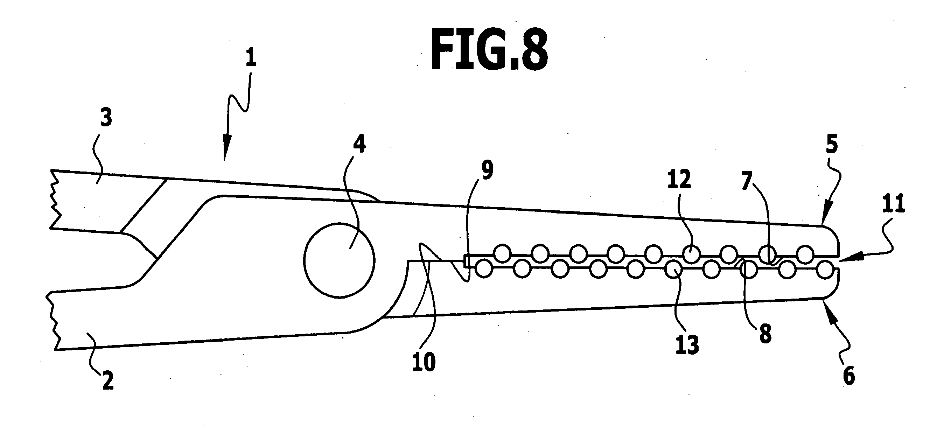

Während bei den Ausführungsbeispielen der Figuren 1 bis 6 die Vorsprünge zapfenförmig ausgebildet sind, werden beim Ausführungsbeispiel der Figuren 7 und 8 Vorsprünge in Form von Querrippen verwendet, die sich über die Breite der Klemmbacken 5, 6 erstrecken und die parallel zu den Grundflächen 7, 8 sowie parallel und im Abstand zueinander verlaufen, im Querschnitt sind die Vorsprünge 12, 13 dieses Ausführungsbeispiels halbkreisförmig ausgebildet und sie sind so gegeneinander seitlich versetzt, daß Vorsprünge 12 des oberen Klemmbackens 5 zwischen Vorsprünge 13 des unteren Klemmbackens 6 eintauchen, wie dies auch aus der Darstellung der Figur 8 deutlich wird. Auch in diesem Falle ist die Anordnung so gewählt, daß sich die Querschnittsabnahme der Vorsprünge des einen Klemmbackens und die Querschnittszunahme der Vorsprünge des anderen Klemmbackens in Richtung von einem Klemmbacken zum anderen im wesentlichen kompensieren, so daß auch in diesem Falle in unterschiedlichen Ebenen des Zwischenraumes 11 im wesentlichen gleiche Flächen für den Stromfluß zur Verfügung stehen.

Die Vorsprünge dienen bei allen Ausführungsformen natürlich auch dazu, das eingeklemmte Gewebe sicher zu erfassen und gegen jegliche Verschiebung zu sichern, dies gelingt insbesondere bei zapfenförmigen Vorsprüngen gut, da bei diesen eine Verschiebung in jede Richtung vermieden werden kann. Eine Vielzahl von derartigen Vorsprüngen führt dazu, daß sich die Klemmkräfte gleichmäßig verteilen, und durch eine geeignete Formgebung der Vorsprünge kann auch erreicht werden, daß das eingeklemmte Gewebe möglichst wenig verletzt wird. Dies ist insbesondere bei am freien Ende ballig ausgebildeten Vorsprüngen gut zu erreichen.

## Patentansprüche

1. Koagulationsklemme (1) für chirurgische Zwecke mit zwei gegeneinander bewegbaren Klemmbacken (5, 6) mit elektrisch leitenden und mit elektrisch isolierenden Bereichen, wobei die elektrisch isolierenden Bereiche durch zum jeweils anderen Klemmbacken (5, 6) weisende Vorsprünge (13, 12) auf einer elektrisch leitenden Grundfläche (8, 7) der Klemmbacken (6, 5) gebildet werden, wobei Anschläge (9, 10) vorgesehen sind, die die Bewegung der beiden Klemmbacken (5, 6) bei deren Annäherung in ihrer angenäherten Schließstellung so begrenzen, dass die Grundflächen (7, 8) im wesentlichen parallel zueinander und im Abstand zueinander angeordnet sind, wobei die Vorsprünge (12, 13) so lang und so angeordnet sind, dass die Vorsprünge (12) eines Klemmbackens (5) in der Schließstellung der Klemmbacken (5, 6) zwischen die Vorsprünge (13) des anderen Klemmbackens (6) eintauchen, wobei der Querschnitt der Vorsprünge (12, 13) im Überlappungsbereich der Vorsprünge (12, 13) der beiden Klemmbacken (5, 6) zum freien Ende der Vorsprünge (12, 13) hin abnimmt, und wobei die Vorsprünge (12, 13) so angeordnet und geformt sind, dass die von den Vorsprüngen (12, 13) freie Fläche in parallel zu den Grundflächen verlaufenden Ebenen in der Schließstellung der Klemmbacken (5, 6) über den gesamten Zwischenraum (11) zwischen den Grundflächen (7, 8) der beiden Klemmbacken (5, 6) im wesentlichen gleich ist, **dadurch gekennzeichnet, dass** die Klemmbacken (5, 6) und die Vorsprünge (12, 13) einstückig ausgebildet sind und die elektrisch leitfähige Grundfläche (7, 8) durch eine Metallfüllung einer oder mehrerer Vertiefungen in den Klemmbacken (5, 6) gebildet wird.

2. Koagulationsklemme nach Anspruch 1, **dadurch gekennzeichnet, daß** die von den Vorsprüngen (12, 13) freie Fläche in parallel zu den Grundflächen verlaufenden Ebenen mindestens so groß ist wie die von den Vorsprüngen (12, 13) eingenommene Fläche.

3. Koagulationsklemme nach einem der voranstehenden Ansprüche, **dadurch gekennzeichnet, daß** die Vorsprünge (12, 13) im Überlappungs-bereich konisch ausgebildet sind.

4. Koagulationsklemme nach Anspruch 1 oder 2, **dadurch gekennzeichnet, daß** die Vorsprünge (12, 13) im Überlappungsbereich ballig ausgebildet sind.

5. Koagulationsklemme nach einem der voranstehenden Ansprüche, **dadurch gekennzeichnet, daß** die Vorsprünge (12, 13) außerhalb des Überlappungsbereiches zylindrisch ausgebildet sind.

6. Koagulationsklemme nach Anspruch 5, **dadurch gekennzeichnet, daß** die Vorsprünge (12, 13) außerhalb des Überlappungsbereiches kreiszylindrisch ausgebildet sind.

7. Koagulationsklemme nach Anspruch 1 oder 2, **dadurch gekennzeichnet, daß** die Vorsprünge (12, 13) als sich über die Breite der Klemmbacken (5, 6) erstreckende Querrippen ausgebildet sind.

8. Koagulationsklemme nach Anspruch 7, **dadurch gekennzeichnet, daß** die Querrippen im Querschnitt halbkreisförmig ausgebildet sind.

9. Koagulationsklemme nach einem der voranstehenden Ansprüche, **dadurch gekennzeichnet, daß** der Abstand von zwei Bereichen der von den Vorsprüngen (12, 13) freien Fläche, die durch einen Vorsprung (12, 13) voneinander getrennt sind, maximal 1,5 mm beträgt.

10. Koagulationsklemme nach einem der voranstehenden Ansprüche, **dadurch gekennzeichnet, daß** die Vorsprünge (12, 13) aus Keramik bestehen.

11. Koagulationsklemme nach einem der Ansprüche 1 bis 9, **dadurch gekennzeichnet, daß** die Vorsprünge (12, 13) aus einem thermoplastischen Kunststoff bestehen.

## Claims

1. Coagulation clamp (1) for surgical purposes with two relatively movable clamping jaws (5, 6) with electrically conductive and with electrically insulating regions, wherein the electrically insulating regions are provided by projections (13, 12) directed towards the respective other clamping jaw (5, 6) on an electrically conductive base surface (8, 7) of the clamping jaws (6, 5), wherein stops (9, 10) are provided, which restrict the movement of the two clamping jaws (5, 6) as they come closer together in their proximate closed position in such a manner that the base surfaces (7, 8) are arranged substantially parallel to one another and spaced from one another, wherein the projections (12, 13) have such a length and are so arranged that in the closed position of the clamping jaws (5, 6) the projections (12) of one clamping jaw (5) project between the projections (13) of the other clamping jaw (6), wherein in the overlapping region of the projections (12, 13) of the two clamping jaws (5, 6) the cross-section of the projections (12, 13) decreases towards the free end of the projections (12, 13), and wherein the projections (12, 13) are arranged and shaped in such a manner that in planes running parallel to the base surfaces in the closed position of the clamping jaws (5, 6) the surface free from projections (12, 13) is substantially uniform over the entire space (11) between the base surfaces (7, 8) of the two clamping jaws (5, 6), **characterised in that** the clamping jaws (5, 6) and the projections (12, 13) are formed in one piece and the electrically conductive base surface (7, 8) is formed by a metal filling of one or more depressions in the clamping jaws (5, 6).

2. Coagulation clamp according to claim 1, **characterised in that** in planes running parallel to the base surfaces, the surface free from projections (12, 13) is at least as large as the surface occupied by the projections (12, 13).

3. Coagulation clamp according to one of the preceding claims, **characterised in that** the projections (12, 13) are conical in configuration in the overlapping region.

4. Coagulation clamp according to claim 1 or 2, **characterised in that** the projections (12, 13) are spherical in configuration in the overlapping region.

5. Coagulation clamp according to one of the preceding claims, **characterised in that** the projections (12, 13) are cylindrical in configuration outside the overlapping region.

6. Coagulation clamp according to claim 5, **characterised in that** the projections (12, 13) are circular cylindrical in configuration outside the overlapping region.

7. Coagulation clamp according to claim 1 or 2, **characterised in that** the projections (12, 13) are configured as cross ribs extending over the width of the clamping jaws (5, 6).

8. Coagulation clamp according to claim 7, **characterised in that** the cross ribs are semicircular in cross-section.

9. Coagulation clamp according to one of the preceding claims, **characterised in that** the spacing of two regions of the surface free from the projections (12, 13) separated from one another by a projection (12, 13) amounts to 1.5 mm at maximum.

10. Coagulation clamp according to one of the preceding claims, **characterised in that** the projections (12, 13) are made from ceramic.

11. Coagulation clamp according to one of claims 1 to 9, **characterised in that** the projections (12, 13) are made from a thermoplastic material.

## Revendications

1. Pince de coagulation (1) destinée à des fins chirurgicales, comprenant deux mors de serrage (5, 6) mobiles l'un par rapport à l'autre et présentant des zones électriquement conductrices et électriquement isolantes, pince
dans laquelle les zones électriquement isolantes sont formées par des protubérances (13, 12) situées sur une surface de base (8, 7) électriquement conductrice des mors de serrage (6, 5) et dirigées respectivement vers l'autre mors de serrage (5, 6),
dans laquelle sont prévues des butées (9, 10) qui limitent le mouvement des deux mors de serrage (5, 6) lors de leur rapprochement dans leur position de fermeture rapprochée, de façon telle que les surfaces de base (7, 8) soient agencées sensiblement de manière parallèle l'une à l'autre et à distance l'une de l'autre,
dans laquelle les protubérances (12, 13) sont d'une longueur et sont agencées de manière telle que les protubérances (12) d'un mors de serrage (5), dans la position de fermeture des mors de serrage (5, 6), s'engagent entre les protubérances (13) de l'autre mors de serrage (6),
dans laquelle la section transversale des protubérances (12, 13), dans la zone de chevauchement réciproque des protubérances (12, 13) des deux mors de serrage (5, 6), diminue en direction de l'extrémité libre des protubérances (12, 13),
et dans laquelle les protubérances (12, 13) sont agencées et formées de manière telle que la surface exempte de protubérances (12, 13), dans des plans s'étendant parallèlement aux surfaces de base, dans la position de fermeture des mors de serrage (5, 6), est sensiblement la même sur l'ensemble de l'espace intermédiaire (11) entre les surfaces de base (7, 8) des deux mors de serrage (5, 6),
**caractérisée en ce que** les mors de serrage (5, 6) et les protubérances (12, 13) sont réalisés d'un seul tenant, et la surface de base (7, 8) électriquement conductrice est formée par un remplissage de métal d'un ou de plusieurs creux dans les mors de serrage (5, 6).

2. Pince de coagulation selon la revendication 1, **caractérisée en ce que** la surface exempte de protubérances (12, 13), dans des plans s'étendant parallèlement aux surfaces de base, est au moins aussi grande que la surface occupée par les protubérances (12, 13).

3. Pince de coagulation selon l'une des revendications précédentes, **caractérisée en ce que** les protubérances (12, 13) sont de configuration conique dans la zone de chevauchement réciproque.

4. Pince de coagulation selon la revendication 1 ou la revendication 2, **caractérisée en ce que** les protubérances (12, 13) sont de configuration bombée dans la zone de chevauchement réciproque.

5. Pince de coagulation selon l'une des revendications précédentes, **caractérisée en ce que** les protubérances (12, 13) sont de configuration cylindrique en-dehors de la zone de chevauchement réciproque.

6. Pince de coagulation selon la revendication 5, **caractérisée en ce que** les protubérances (12, 13) sont de configuration cylindrique circulaire en-dehors de la zone de chevauchement réciproque.

7. Pince de coagulation selon la revendication 1 ou la revendication 2, **caractérisée en ce que** les protubérances (12, 13) sont réalisées en tant que nervures transversales s'étendant sur la largeur des mors de serrage (5, 6).

8. Pince de coagulation selon la revendication 7, **caractérisée en ce que** les nervures transversales présentent, en section transversale, une configuration en forme de demi-cercle.

9. Pince de coagulation selon l'une des revendications précédentes, **caractérisée en ce que** la distance d'espacement de deux zones de la surface exempte de protubérances (12, 13), qui sont séparées par une protubérance (12, 13), est d'une valeur au maximum de 1,5 mm.

10. Pince de coagulation selon l'une des revendications précédentes, **caractérisée en ce que** les protubérances (12, 13) sont réalisées en céramique.

11. Pince de coagulation selon l'une des revendications 1 à 9, **caractérisée en ce que** les protubérances (12, 13) sont réalisées en un matériau synthétique thermoplastique.
